# EUROPEAN PATENT APPLICATION

(11) **EP 4 817 861 A1**
(43) Date of publication of application: **07.10.2026**
(21) Application number: 26160583.6
(22) Date of filing: 25.02.2026
(51) Int. Cl.: B32B 3/28, A61F 13/49, B32B 5/02, B32B 7/14, B32B 25/10, B32B 25/14, B32B 27/12

(54) **ELASTIC LAMINATE AND METHOD FOR PRODUCING AN ELASTIC LAMINATE**

(30) Priority: 31.03.2025 CN 202510392099
(71) Applicant: Nitto Denko Corporation, Ibaraki-shi, Osaka 567-8680 (JP)
(72) Inventor: Choi, Hong Lim, Seoul (KR); Lin, Bao Hui, Taicang Jiangsu (CN)
(74) Representative: Andrejewski - Honke Patent- und Rechtsanwälte Partnerschaft mbB

(57) **Abstract**

The invention relates to an elastic laminate with at least one nonwoven top layer and an elastic film bonded to it. The elongation at a load of 700 g according to DIN ISO 527 is at least 150% in the machine direction and at least 100% in the cross-direction.

## Description

The invention relates to an elastic laminate or a top layer made of a nonwoven and an elastic film bonded to it.

Such laminates are known from the prior art and are widely used in hygiene products because they have a soft surface and can also provide a certain degree of elasticity. For example, corresponding laminates are used as diaper ears or also as disposable underwear because they are inexpensive to manufacture and at the same time provide a high degree of comfort.

However, to enable a corresponding application, it should be noted that the at least one top layer made of nonwoven, in contrast to the elastic film, usually has only a very low stretchability, so that the elasticity of the elastic film must also be released with regard to the elastic laminate by means of suitable manufacturing processes.

Various methods are known from practice to create elasticity in the cross direction in a laminate made of nonwoven and elastic film. For example, the so-called neckbonding is known, wherein different embodiments of this method are explained in the documents EP 0 707 106 B1, EP 1 458 565 B1 and US 7 008 496 B2.

In the case of neck-bonding, the nonwoven is guided at an increased draw-off speed after being unwound. This causes the nonwoven to be stretched in length and to constrict. This results in folds that are arranged next to each other in the cross-direction and aligned in the machine direction. The nonwoven must have a corresponding fiber structure so that the fibers align during necking and can be erected. The nonwoven folded in this way is then joined to an elastic film.

US 2006/003656 A1 teaches a design in which the entire laminate is additionally stretched by means of ring rolling after the nonwoven web has been laminated with the elastic film. Although this techniques have proven successful in principle, it has also been shown that setting certain elasticity parameters is problematic, since the exclusive folding by constricting the nonwoven web means that the material is significantly less stressed at the edges than in the center of the nonwoven web, resulting in an uneven folding across the width of the nonwoven web. This is particularly problematic when, in accordance with a conventional process, the nonwoven web is folded in the cross-direction, resulting in cross-direction stretchability of the laminate. At the same time, the elasticity parameters can only be adjusted to a limited extent because the bonding points are independent of the folds in the nonwoven. Accordingly, the elasticity parameters can vary to a certain extent across the width and/or length of the nonwoven web. Anotherway of enabling the formation of an elastic laminate is so-called stretch bonding. For this, the nonwoven web is supplied essentially untreated, while the elastic film is stretched in the machine direction or in the cross-direction to a desired extent. The nonwoven web is joined to the elastic film in a stretched state of the elastic film, whereby the elastic laminate then forms folds due to the elastic film resetting. Accordingly, the folds are not actively introduced into the nonwoven web, but are formed exclusively by the elastic film resetting.

The formation of the bonding points occurs accordingly in the stretched state of the elastic film, so that a uniform bonding pattern can be achieved in the stretched state.

Such a design has the advantage that the elasticity and also the wrinkling in the stretched state are much more defined than in the previously described neck-bonding. However, in such a process, the elasticity parameters are almost exclusively influenced by the pattern of the bonding points.

Although the methods described so far have proven themselves in principle, they result in the elastic laminate having high elasticity parameters either in the machine direction or in the cross-direction, while the material offset to this direction has only very low extensibility and, as a rule, hardly any elastic recovery properties. In view of this, the laminates are often only suitable to a very limited extent for hygiene products that need to adapt as well as possible to different parts of the body.

In view of this, the aim of the invention is to provide a laminate that can be used flexibly for a variety of products and at the same time has a good feel compared to the products known to date.

The object and solution of this task is an elastic laminate according to patent claim 1. Accordingly, the elongation at a load of 700 g according to DIN ISO 527 is at least 150% in the machine direction (MD) and at least 100% in the cross-direction (CD). Particularly preferred is an elongation at 700 g in accordance with DIN ISO 527 of at least 200% in the machine direction and/or at least 140% in the cross-direction.

This refers to a test procedure in accordance with DIN ISO 527 in which a 50.8 mm wide (2 inch) sample is loaded with a weightof 700 g. The change in length (ΔL) determined in such a measurement can be compared with the original length (L0), where an elongation of 100% means a change in length ΔL by the original length L0. Accordingly, a material with an initial length of 10 mm would have an extended length of 20 mm as a result of a load of 700 g.

It is also particularly preferred if the elastic laminate not only has a high degree of elasticity in the machine direction and in the cross-direction. Rather, the elastic laminate is characterized by high extensibility regardless of direction. In particular, it is envisaged that the elongation at a load of 700 g according to DIN ISO 527 in a diagonal direction arranged at 45° to the machine direction is at least 100%, and is particularly preferably at least 150%. Furthermore, the strain at a load of 700 g according to DIN ISO 527 in a diagonal direction arranged at 30° to the machine direction and/or cross-direction can be at least 100%, preferably at least 150%. Accordingly, it is apparent that the laminate can be used for various types of hygiene products, essentially regardless of the alignment, and that, at the same time, the high isotropic stretchability enables good adaptability and high wearing comfort.

In order to develop such elasticity in an elastic laminate, it is particularly intended that the elastic film and the at least one top layer are attached to one another at a large number of spaced-apart bonding points. Accordingly, there is no full-surface bonding between the nonwoven and the elastic film, with the bonding points at the same time being of great importance with regard to the elasticity of the laminate.

Accordingly, such an elastic laminate can be produced, for example, by a process comprising the following steps:
a) feeding at least one nonwoven web and one elastic film web in a machine direction,
b) forming a plurality of folds by pre-folding the at least one nonwoven web in cross-direction while supporting and guiding the nonwoven web along a longitudinal section,
c) stretching the elastic film web in machine direction, and
d) forming the elastic laminate by joining the stretched elastic film web to the at least one nonwoven web that has been folded.

In this context, a longitudinal section isa section along the machine direction over which the nonwoven webs are guided during the folding process. In contrast to ring rolling, the nonwoven web is therefore guided essentially force-free along fins extending in the longitudinal direction, the fins engaging in one another in the same way as in ring rolling, thus enabling the nonwoven to be folded. However, there is no point contact between two rollers, but a linear contact along a longitudinal section, whereby the longitudinal section must be sufficiently long to allow the formation of folds, taking into account the web speed.

The axial longitudinal section can beat least 500 mm, preferably at least 600 mm, long. It is also important that the nonwoven web is guided between the fins without force, so that the width of the nonwoven web is significantly reduced in the course of folding, while in ring rolling the nonwoven web is held at the lateral edges. This means that the nonwoven web is folded, but there is no stretching or overstretching of the material in the cross-direction. At the same time, the longitudinal support ensures that the material has sufficient time to form uniform folds across the entire width of the nonwoven web.

In this manufacturing process, elasticity in the cross-direction and in the machine direction is realized in different ways. While elasticity in the cross-direction is achieved by folding the nonwoven, stretchability in the machine direction is achieved by stretching the elastic film web and then joining the folded nonwoven web to the elastic film. Accordingly, a combination of stretch bonding and a modified ring rolling process is carried out to a certain extent, although itshould be emphasized again at this point that the cross-direction folding process differs significantly from the ring rolling process. However, in both cases, the folding is done by resetting the elastic film.

Since the top layer made of nonwoven is only folded but not stretched in cross-direction, the bonding with the elastic film occurs in a substantially unaffected state, so that when the laminate is stretched in the cross-direction, only the elastic film is stretched in the areas between the bonding points, while the laminate itself remains unaffected at the bonding points. Consequently, the elasticity is largely determined by the bonding points.

The result of integrating such a manufacturing process is that, in a state stretched in the machine direction, in which the bonding points are also imprinted, the top layer of nonwoven has essentially uniform folding, with the folds running along the machine direction. However, the recovery of the elastic film then causes the regular formation of the folds to be discarded, so that instead of a regular formation of folds, a top layer with a hilly surface is formed. Accordingly, the elastic laminate has a significantly different surface structure from the previously known laminates, which, in addition to the irregular structure, also has a comparatively dense feel.

In addition, it is preferred that the surface weight at the bonding points is greater than the surface weight between the bonding points. Such a design can result in particular during production using the method according to the invention, since the bonding points have a greater width than the folds being created during the process step b).

In contrast to the solutions known to date, the process described above in particular allows very fine micro-folds to be formed in the cross direction. This is achieved, for example, by stretching the nonwoven web in the machine direction after pre-folding. This stretching not only has the effect of refining the coarse pre-fold structure previously formed in the course of the longitudinal support.

The result of this process is that a very fine micro-folded structure is formed in the nonwoven web, with the micro-folds being significantly narrower than the bonding points. In the course of bonding, a large number of micro-folds can be included in the cross direction in a bonding point. For example, between 6 and 14 folds, in particular between 8 and 12, can be included by just one bonding point. This has the consequence that the material is further compressed during bonding by the folding at the bonding points, so that the elasticity is determined to an even greater extent by the bonding points. Thus, for example, a greater stretch and also a highertensile strength can be achieved with a small number of integrated folds. By contrast, a larger number of integrated folds results in improved resilience from the stretched to the unstretched state. Depending on the application, the elasticity of the laminate can therefore be significantly influenced, in particular by the design of the bonding points.

This also has the consequence that the stretching behaviorof the elastic film is largely defined by the bonding points. Thus, the tensile strength according to DIN ISO 527 in the machine direction is at least 35 N, preferably at least 40 N, and/or in the cross-direction at least25 N. Compared to the known elasticlaminates, the laminate according to the invention is characterized by a high tensile strength, for which the particularly stable bonding points are crucial.

In addition to a largely unaffected nonwoven top layer, the accumulation of material due to the integration of a large number of folds at the bonding points causes these to form a kind of anchor point, which together stabilize the elastic laminate. In the course of stretching, the elasticity of the film is initially utilized, depending on the direction, until the top layer lies largely flat. In this case, the elastic film is stretched until the nonwoven top layer lies essentially flat and now forms a kind of barrier to further stretching of the elastic laminate. This resistance arises from the fact that, on the one hand, the nonwoven web is present uninfluenced and thus in a non-thinned form between the bonding points and, on the other hand, the accumulation of material in the area of the bonding points introduces additional stability into the elastic laminate.

This ensures that products made of the elastic laminate can be stretched particularly well in essentially any direction. At the same time, it also prevents the laminate from tearing too easily when an external force is applied.

In addition to the elasticity of the elastic laminate, the so-called permanent set is also important, which provides information about how the elastic laminate is permanently extended after an initial stretch. To do this, a material sample with a width of 50.8 mm (2 inches) is clamped again and stretched 70% in the cross-direction and/or 150% in the machine direction. The material is then returned to its unstretched or force-free state, with the length difference in relation to the original clamping length defining the permanent set. Based on such a measurement, the permanent set after an initial stretch of 150% in the machine direction is max. 30% and/or after an initial stretch of 70% in the cross-direction is max. 30%.

Particularly preferred is a permanent set of max. 20% after an initial stretch of 150% in the machine direction and/or max. 20% after an initial stretch of 70% in the cross-direction.

A laminate of this kind, which on the one hand allows a high degree of stretch in the cross- and machine direction and at the same time has a low permanent set, is suitable for a wide range of products. Particularly when used in hygiene products such as diapers, the use of such laminates ensures that the high elasticity provides a high level of comfort and a secure closure. At the same time, the low permanent set allows the diapers to be closed several times or subjected to high loads without causing problematic stretching. In this context, the present invention relates not only to elastic laminates but also to products made from such laminates, in particular hygiene products.

Although it is sufficient in the context of the invention to bond only one top layer of nonwoven to the elastic film, a preferred embodiment provides that the elastic film is arranged between at least two top layers of nonwoven. Accordingly, the elastic film is enclosed between the two nonwoven top layers, both top layers having a hilly surface in an unstretched and thus force-free state. Accordingly, the laminate has a soft and fleecy surface on both sides, which results in a better feel for the user, particularly in the design of hygiene products.

On the basis of such a design, the top layers and the elastic film can be directly bonded to one another. This can be done, on the one hand, by applying dots of adhesive, e.g. from a hot-melt adhesive. However, a design in which the bonding points are created by melting the elastic laminate is particularly preferred. This can be achieved, for example, by thermal spot welding or ultrasonic welding. In the case of thermal spot welding, the pattern of the bonding points is imprinted by hot rollers that have corresponding protruding sections in the form of the bonding pattern. The melting is then achieved by the introduction of thermal energy.

In ultrasonic welding, pressure is applied to the laminate at the intended bonding points via a so-called anvil roller with protrusions and ultrasonic sonotrodes are used to melt the top layers and/or the elastic film.

Regardless of the type of welding, it is theoretically possible that only the elastic film melts to a certain extent and the two nonwoven top layers are joined together by the molten material. At the same time, however, it is also conceivable that the application of pressure and energy in combination with the stretching of the elastic film causes the elastic film to tear at the bonding points and that the nonwoven top layers are then directly bonded to one another, at least partially or completely, at the bonding points. This naturally presupposes that the nonwoven is formed from a material which consists at least partially of thermoplastic filaments and/or fibers. In particular, filaments and/or fibers made of a polyolefin, e.g. polypropylene (PP) or polyethylene (PE), are suitable for this purpose.

The formation of the bonding points can be decisive for the elastic behavior of the laminate, with the size of the bonding points and their distance from one another being particularly important. According to a preferred embodiment, the bonding points are arranged in longitudinal rows running in the machine direction and in transverse rows running in the cross direction. In this regard, the arrangement in the cross direction is particularly important, since the material is essentially only elastic in the cross direction. The bonding points can be round, oval, square or rectangular, with the width in the cross direction being particularly crucial for how many folds can be fixed at the bonding point. A particularly preferred embodimentin this context provides that the width of the bonding points in the cross direction is between 0.8 and 2 mm, in particular between 1 and 1.6 mm. The length in the machine direction is preferably between 0.1 and 0.5 mm. Another important factor with regard to elasticity is not only the width of the bonding points and thus the number of possible integrated folds, but also the distance between the bonding points in the cross direction. This distance is preferably between 1 mm and 2.5 mm, in particular between 1.4 and 2 mm. The ratio between the distance of the bonding points in the cross direction and the width of the bonding points also determines the ratio between the completely free folds and the folds that are fixed in the bonding points. The elasticity of the laminate in the cross direction can therefore be adjusted by specifically selecting this ratio, so that, in contrast to the previously known laminates, which were manufactured using the neck-bonding or stretch-bonding method, both the folding and the selection of the pattern for the bonding points are of crucial importance.

A further development of the invention also provides that the elastic film has an elastic film layer and at least a first non-elastic outer layer coextruded with the elastic film layer. Preferably, a first and a second non-elastic outer layer are coextruded with the elastic film layer, the elastic film layer being arranged between the first and the second outer layer. In principle, various materials are suitable for forming the outer layers and the elastic film layer. The elastic film layer is preferably made from polyolefin elastomers or styrene block copolymer compounds. This styrene block copolymer compounds may, for example, be a styrene-isoprene-styrene block copolymer (SIS), a styrene-butadiene-styrene block copolymer (SBS), a styrene-ethylene-butylene-styrene (SEBS) or a styrene-ethylene/propylene-styrene block copolymer (SEPS). Furthermore the styrene block copolymer compounds may be mixed with a polyolefin, i.e. a polyolefin elastomers, or a polyolefin mixture. Particular preference is given to the styrene block copolymer being a styrene-ethylene-propylene-styrene block copolymer (SEEPS), SEEPS having the special feature that it can be mixed particularly well with polyolefins. Accordingly, processing agents such as mineral oil can be dispensed with.

The second polymer componentis preferably present in an amount between 25 and 90% in the elastic film layer. In addition to a polyolefin ora polyolefin mixture, polystyrene or processing agents may also be provided. The processing agents may in particular be mineral oil.

In a preferred embodiment, the first and/or second outer layers are formed from a polyolefin as the main constituent. In this context, "main constituent" means that the polyolefin is present in the first and/or second outer layer in a proportion of at least 30% by weight. It is particularly preferred for at least 45 wt.% of the polyolefin to be present in the respective first and/or second outer layer, and particularly preferred for at least 50 wt.% to be present. The polyolefins of the outer layers and/or the elastic film layer are preferably polyethylene, polypropylene or mixtures thereof. In the case of polyethylene, this is in particular a low-density polyethylene (LDPE) or a linear low-density polyethylene (LLDPE). The first and/or second outer layer may also contain 2 to15% fillers such as chalk or talc.

In this context, a particularly preferred embodiment provides that the ratio of the thickness of the elastic core layer to the thickness of the first and/or second outer layer is between 6:1 and 15:1, in particular between 7:1 and 12:1. In particular, it is envisaged that the first and/or second outer layer has a thickness between 1 µm and 5 µm, preferably between 1.5 and 4 µm, particularly preferably between 2 µm and 3.5 µm. By contrast, the elastic core layer has a thickness between 15 and 40 µm, particularly between 20 and 30 µm.

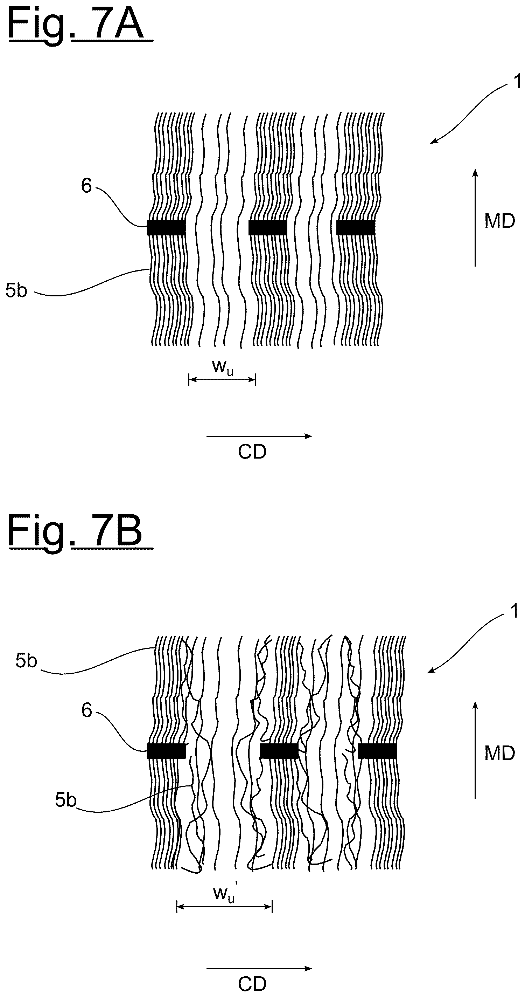

A preferred embodiment of the invention provides that an open hole force (OHF) is between 30 and 140 N. This parameter is determined using a measuring device, which is explained in more detail below in connection with Fig. 7. When measuring this parameter, a sample is fixed on a sample holder, which hasan an internal diameter D_{H} of 35 mm. Then the laminate is stressed with a mechanical finger, the size of which is based on a human finger. The mechanical finger is cone-shaped and has a base area with a diameter D_{T} of 20 mm and a length Z_{T} of 15 mm. At the tip, the mechanical finger is rounded with a radius R_{T} of 5 mm. This mechanical finger has a surface material made of neoprene rubber having a hardness between 5 and 20 HRC. The mechanical finger is then guided in the direction of the specimen holder and presses the elastic laminate over the tip into the open area of the specimen holder formed by the inner diameter. The speed at which the mechanical finger is inserted is 1260 mm/min. The force is then recorded until the breaking elongation limit is reached, with the maximum measured force corresponding to the puncture hole force (OHF).

The invention further relates to a method for producing an elastic laminate , in particular according to patent claim 17, comprising at least the following steps:
a) supplying at least one nonwoven web and one elastic film web in a machine direction,
b) forming a plurality of folds by pre-folding the at least one nonwoven web in cross-direction, preferably while it is being supported along a longitudinal section,
c) stretching the elastic film web in machine direction, and
d) forming the elastic laminate by joining the stretched elastic film to the at least one nonwoven web that has been folded.

The formation of the folds during the supported guidance takes place over a longitudinal section with a length between 500 mm and 1,500 mm, in particular between 600 mm and 1,200 mm. In contrast to the so-called ring-rolling method, the formation of folds during the longitudinal section occurs in a static manner, whereby the nonwoven web is guided between a series of upper and lower interlocking fins. The distance between the fins and the extent to which the fins engage with each other are decisive for the folds that are formed as a result.

In particular, the distance between two consecutive fins in the cross-direction is between 5 mm and 50 mm. The degree to which the upper and lowerfinsengage with each other is preferably between 5 mm and 40 mm. The upper and lower rows of fins are part of a folding device, whereby the nonwoven web, after leaving the fins, has a folding which is often too coarse for the intended laminate. In view of this, it is necessary to convert the rough folding into a finer micro-folding. Accordingly, the folding in step b) during the supported guidance merely forms a kind of pre-folding, with the fully formed folds having a significantly smaller width and height than the pre-folds.

This type of pre-folding, in which the nonwoven web is guided over fins in a straight line and without tension, also differs significantly from the folding of a material web in a roller gap or by wrapping around a profiled roller.

In order to achieve such a micro-fold, the non woven web, which has already been pre-folded, can then be stretched in the machine direction by at least 10% during step b). This stretching must be carried out after the pre-folding, which ensures that the folding takes place evenly over the cross-direction width of the nonwoven web. If the nonwoven web were stretched during the folding process, the folds in the center of the nonwoven web would be significantly larger than at the edges, since the material could not be fed evenly into the area between the fins and would therefore be stretched more in the center. The subsequent stretching transforms the pre-folds into micro-folds, while the width of the nonwoven web does not change. At the same time, the application of the stretch also stabilizes the folds in the nonwoven web, so that the folded nonwoven web can then be joined to the elastic film.

It is particularly preferred that the nonwoven web is stretched between 10% and 30%, especially between 15% and 25%. The stretching can be achieved by rollers that run at different speeds. However, it must be ensured that the stretching only stabilizes and reshapes the folds, whereby no significant thinning of the nonwoven web occurs.

For the formation of the elastic laminate, it is also of crucial importance that the nonwoven web and the elastic film web are precisely dimensioned with respect to each other in terms of their width in the supplied state, whereby both the nonwoven web in the course of being folded and the elastic film in the course of being stretched in the machine direction are reduced in width, which must be taken into account accordingly.

On this basis, it is preferably provided that the nonwoven web that is folded has a width in the cross-direction that is between 40% and 90% of the non woven web that is unfolded. Accordingly, the width is reduced by 10% to 60% in the course of pre-folding in step b). Furthermore, the width of the folded nonwoven web is between 80% and 400% of the width of the unstretched elastic film. In this case, the ratio depends in particularon the extent to which the elastic film is reduced in width during stretching in the machine direction due to the materials used.

Regardless of this, the elastic film is stretched between 150% and 500% in step d). The width of the stretched elastic film can be between 10% and 50% of the unstretched elastic film.

A further development of the invention provides that the elastic film and the at least one nonwoven web are bonded to one another at a plurality of bonding points spaced apart from one another in step d). As already explained, this can be done preferably by ultrasonic welding or by thermal spot welding. The formation of the bonding points is decisive for the elastic behaviorof the laminate, with the size of the bonding points and their distance from one another being particularly important.

According to a preferred embodiment, the bonding points are arranged in longitudinal rows running in the machine direction and in transverse rows running in the cross direction. In this context, a particularly preferred embodiment provides that the width of the bonding points in the cross direction is between 0.8 mm and 2 mm, in particular between 1 mm and 1.6 mm. The length in the machine direction is preferably between 0.1 mm and 0.5 mm. In this case, bonding points directly adjacent to one another in a longitudinal row and/or cross-direction row can also have different sizes. Furthermore, it is also conceivable that the bonding points of two cross-direction longitudinal rows adjacent to one another in the cross-direction are arranged offset to one another in the machine direction.

The location and design of the bonding points is also crucial for the fold structure in the laminate. The folds that run from the bonding points in the machine direction are usually significantly narrower than the folds that are not integrated along the machine direction via a bonding point. It should be noted that the bonding points are introduced during production in a folded state of the nonwoven web, which has a large number of micro-folds arranged next to each other. By introducing the bonding points, the micro-folds are then fixed at the bonding point, while outside the bonding points the micro-folds essentially form a macro-fold together, caused by at least a partial unfolding. Accordingly, the folds that are integrated in a cross-direction in a bonding point are micro-folds, while the folds between the bonding points are macro-folds. In this context, a micro-fold is a fold with a width of less than 1 mm or less, respectively less than 0.5 mm, while a macro-fold has a width of more than 0.5 mm, in particular more than 1 mm. The micro-folds formed in step b) can have a cross-direction width up to 1mm. Furthermore, the micro-folds usually don't have such a defined fold formation as the macro-folds, which comprise a more random and non-linear fold structure.

Although the folds between the bonding points are macro-folds, a close examination also reveals the micro-fold structure. Usually, the micro-folds are arranged relatively evenly along the cross-direction before the bonding points are applied. However, it is also possible that several micro-folds form a certain macroscopic pre-structure, which has an influence on the structure of the laminate after the bonding points have been applied.

The ratio between the distance between the bonding points and the width of the bonding points also determines the ratio between the fully exposed folds and the foldsthat are fixed in bonding points. Thus, a preferred embodimentprovides that in step d), the bonding points each enclose a plurality of micro-folds, in particular between 6 and 14 folds. The elasticity of the laminate in the cross-direction can therefore be adjusted by specifically selecting the distance and size of the bonding points, since this also determines how many folds are included in the bonding points.

According to a preferred further development of the process, in a step e) following step d), the elastic laminate is activated in the cross-direction. This is done, for example, in the ring-rolling process already explained. The activation can cause the formation of a hole structure in the bonding points or reinforce an already formed hole structure. This structure is produced in particular by melting and/or tearing of the elastic film web in the course of forming the bonding points, whereby the openings of the hole structure are directly adjacent to the bonding points and may even completely enclose them. Accordingly, activation in the cross-direction can, for example, improve air permeability.

Furthermore, activation in the cross direction can also be used specifically to influence the elasticity of the laminate in the cross direction. In this context, it has already been explained that a large numberof micro-folds can be trapped in the bonding points during the formation of the bonding points. As a result of the subsequent activation of the laminate in the cross direction with a high degree of lateral strain, individual micro-folds can then be pulled out of the bonding point Furthermore the material of the micro-folds can orient, fray and/or partially tear. According to that the number of trapped micro-folds in the bonding points is reduced, which leads to the fact that in particular the folds directly adjacent to the bonding points have a larger cross-section in height and/or width, since the pulled-out micro-folds can merge with the adjacent folds. In the course of activation in the cross direction, between two and four micro-folds can be released from individual or all bonding points. In this respect, activation in the cross direction represents a further means of adjusting the elasticity in the cross direction in a targeted manner. Another aspect for the irregular formation of the folded structure is that the independent arrangement between the bonding points and the pre-fold structure of the nonwoven web also causes folded areas of the nonwoven webto be enclosed in the bonding points. This can also lead to altered cross-sections in the finished laminate.

In addition, the micro-folds created in step b) can prevent the lateral strain from destroying the fiber structure of the nonwoven web in the course of the subsequent activation step d), since the areas between the bonding points are first unfolded, so that the material is pulled smooth to a certain extent. Only after this smoothing can overstretching occur, causing damage to the fiber structure.

In a final step f), the elastic laminate can then be wound up.

In addition, at least two nonwoven webs can be supplied and folded, whereby the elastic film web is inserted in a state stretched in the machine direction between the folded nonwoven webs and bonded to them at the bonding points. Usually, the two nonwoven webs are folded in an identical manner, but it is also conceivable to use differently folded nonwoven webs for the two sides of the laminate, so that a different feel is obtained on the two sides of the laminate.

To form the bonding points, it may also be useful for the nonwoven webs to be directly connected to each other through holes in the elastic film. This requires that the nonwoven webs be formed at least in part from a thermoplastic material. In addition, however, it is also conceivable that the material of the elastic film remains at the bonding points, which also acts as a kind of hot-melt adhesive.

The invention will be explained in more detail below with the help of exemplary drawings and examples. The following show:
- Fig. 1: an elastic laminate according to the invention in a sectional view
- Fig. 2: a schematic representation of the manufacturing process according to the invention for forming a laminate according to Fig. 1
- Fig. 3: a schematic representation of a folding device for folding the nonwoven webs
- Fig. 4A, 4B: a schematic representation of the top layers and the elastic film in an unstretched and stretched state and
- Fig. 5A to 5C: schematic representation of formation of folds
- Fig. 6A to 6D: various patterns for the formation and arrangement of the bonding points
- Fig. 7A, 7B: schematic representation of the influence of subsequent ring rolling on material density during an in MD direction stretched state

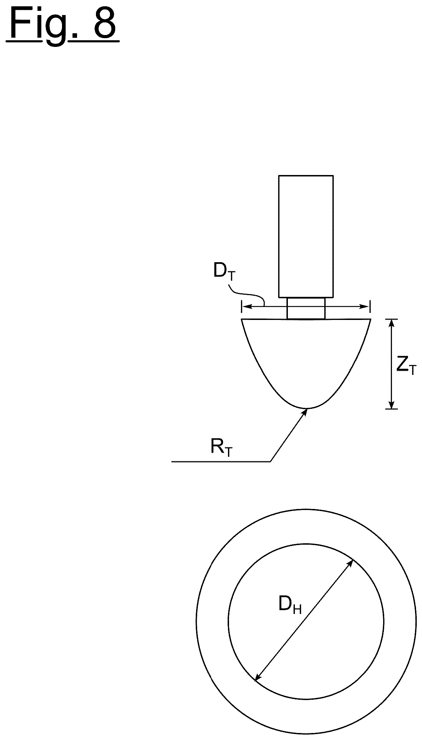

- Fig. 8: schematic representation of the open hole force measurement

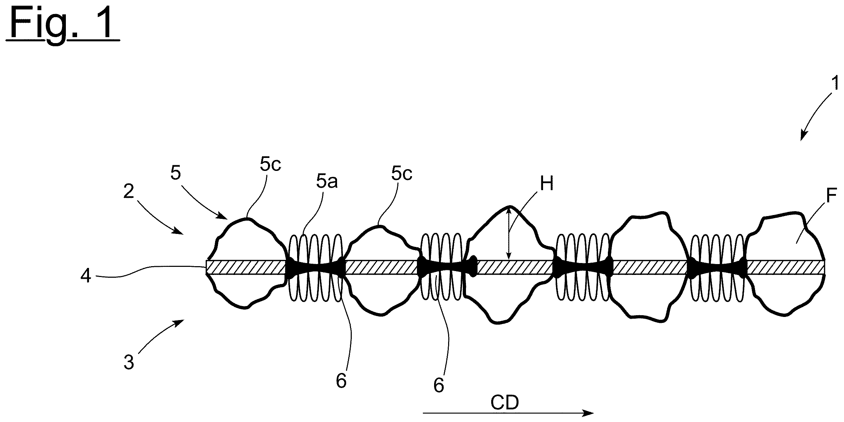

Figure 1 shows an elastic laminate with a first and a second nonwoven top layer 2, 3 and an elastic film4 arranged between the top layers 2, 3. The representation shown in Figure 1 shows that the laminate 1 is joined along a cross-direction CD, which is arranged perpendicular to the machine direction MD in the course of the manufacturing process.

The top layers 2, 3 have a plurality of folds 5 running in the machine direction, with the elastic film 4 being bonded to the top layers 2, 3 at a plurality of bonding points 6 spaced in the machine direction MD and in the cross-direction CD. With regard to the illustration in Figure 1, it should be noted that the elastic laminate 1 shown there is in a state stretched in the machine direction MD, so that the formation of the folds 5 in the cross-direction CD can be seen more clearly. In a non-stretched state in both the cross-direction CD and the machine direction MD, the folds 5 overlap, resulting in an irregular surface. For reasons of better representability, the laminate 1 is therefore shown in a stretched state in the machine direction MD, with the folds 5 then running essentially along the machine direction MD.

As one can see the folds 5 between the bonding points 6 in an unstretched state along the cross-direction are shaped as macro-folds 5c, which have a different cross-section. As one can see in Fig. 1 the folds 5 differ in their shape and/or form, wherein the form of the micro-folds 5c can be described with respect to their area F or their height H. As a consequence the laminate 1 has an irregular corrugated surface, which is formed on both sides by the top layers 2, 3.

Furthermore, in a state stretched in the cross direction CD, in which the top layers 2, 3 are essentially free of macro-folds 5c in the stretching direction, the surface weight at the bonding points 6 is greater than the surface weight between the bonding points 6. This is essentially achieved by two different aspects.

On the one hand, the top layers 2, 3 are folded. However, no material-technical processing of the nonwoven webs 8 from which the top layers 2, 3 are formed is carried out. In the present case, however, the top layers 2, 3 are laid exclusively in micro-folds 5b without any material processing, so that the nonwoven or the top layers 2, 3 have an identical surface weightin an unstretched state regardless of the position in the cross direction CD. However, when stretched, the elastic film 4 becomes thinner.

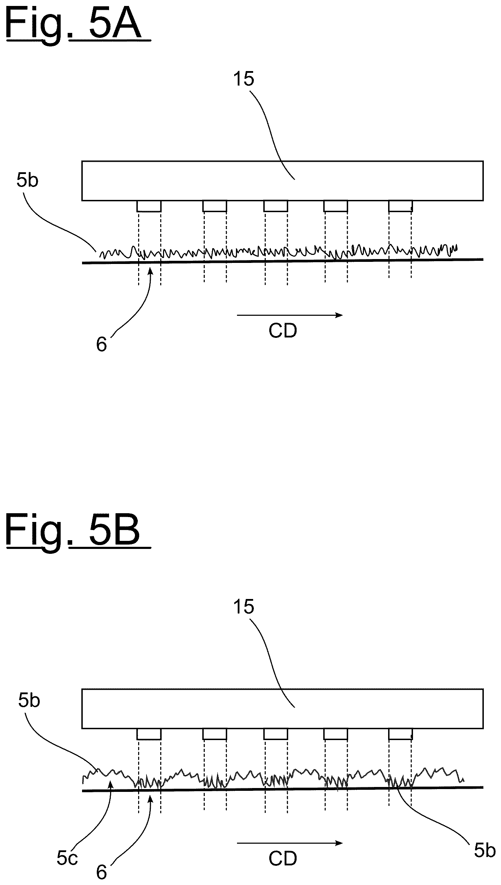

A second, even more decisive aspect is that the bonding points 6 are larger than the width of the micro-folds 5b in an area of the bonding points 6. This effect will be described later with respect to the manufacturing process. However the width of the micro-folds 5b in the bonding point6 can be less than 0,5 mm. These micro-folds 5b, which are shown in more detail in Fig. 5B. The width of the bonding points 6 is between 1 mm and 6 mm. As a result, several micro-folds 5b are usually enclosed at each of the bonding points 6. In the example shown in Fig. 1, two or three folds 5 are schematically integrated into each bonding point 6. However, this is only a schematic representation to better illustrate the facts. As a rule, between 6 and 14 folds 5 are arranged within a bonding point6.

By integrating several micro-folds 5b into the bonding points 6 and stretching the laminate 1 in a cross direction afterwards, the material density or the surface weight is significantly increased compared to the areas Wu' between the bonding points 6, which is exemplary shown in Fig. 7B. On the basis of the representation in Fig. 1 and 7, it is also clear that due to the melting of the top layers 2, 3 at the bonding points 6 these bonding points 6 are more or less free of any fold 5. However, the micro-folds 5c being trapped at the bonding points 6 run across the machine direction MD and are therefore visible behind the bonding points 6. For that reason, Fig. 1 shows the micro-folds 5c at the bonding points 6, wherein these micro-folds 5c start in reality behind the bonding points 6 in machine direction 6.

The measures described above make it possible to design the surface weight at the bonding points 6 to be at least 10% greater than the surface weight between the bonding points 6 in the stretched state. The surface weight of the entire elastic laminate can be between 70 and 110 gsm on average, with the surface weight being largely determined by the top layers 1, 3.

### Top layers 2, 3

Nonwovens are used in particular for the top layers. These nonwovens can be spunbond, spunlace, carded, airlaid or meltblown nononwoven. Spunbond nonwovens are preferred. In the spunbond process, so-called spun fibres, which are spun as continuous filaments from a melt or solution, are stretched in the longitudinal direction. The individual fibres are then fused using suitable measures to form a nonwoven from an initially loose arrangement of filaments.

Furthermore, it is advantageous that the fibers are usually formed from a thermoplastic material, so that the filaments can also be partially fused together by the application of heat. To do this, the nonwoven web can be passed through an oven, for example, in which hot air is applied to the nonwoven web. The hot air penetrates the nonwoven web and causes partial melting of the filaments. This does not significantly destroy the structure of the filaments. Rather, the surface melts, allowing the filaments to bond with each other. Furthermore, it is also conceivable to bond the nonwoven webs together at discrete points using the so-called thermoembossing process. In this process, fusing points are created at discrete points by the targeted application of pressure and temperature, with the size and number of the fusing points being decisive for the strength of the nonwoven. Instead of fibres that are entirely made of a thermoplastic material, it is also possible to combine different materials for the fibres. One possibility are so-called bi-component fibres which are made of two different materials, wherein a first polymer forms a core and a second polymer forms a coat, wherein it is sufficientwhen only the second polymer is chosen form a thermoplastic polymer. Alternatively fibre mixtures can be used, wherein thermoplastic fibres and non-thermoplastic fibres are mixed. In this case even natural or semisynthetic fibres can be used.

The top layers 2, 3 made of nonwoven are preferably formed from filaments, which in turn consist of polypropylene or polyethylene. In the case of polyethylene, low-density polyethylene (LDPE)or linear low-density polyethylene (LLDPE) are preferred.

Of course, it is also conceivable that other types of fibers or filaments not made of a thermoplastic material are added to the nonwoven top layers, 2, 3. These may be natural fibers such as cotton fibers. However, the main component of thermoplastic filaments ensures that the cover layers 2, 3 can be treated with heat and, in particular, can be fixed to the elastic laminate 1 in a subsequent bonding process with the elastic film 4. A main constituent is understood to mean that the continuous filaments of thermoplastic material are present in the top layers 2, 3 in each case in an amountof at least 30% by weight, in particular at least 50% by weight.

### Elastic film 4

The elastic film consists of an elastic core layer and at least a first non-elastic outer layer coextruded with the elastic core layer.

The non-elastic outer layer is used in particular to produce the elastic film. The non-elastic layer ensures that the co-extruded material is interlocked during coextrusion, which considerably simplifies the handling of the elastic film. In addition, the non-elastic outer layer also serves to maintain a web tension. It should be noted that the films are usuallytransported in a machine direction (MD) during the manufacturing and processing, whereby a web tension must be applied to the elastic core layer in the machine direction. If the elastic film were formed exclusively by the elastic core layer, this web tension would cause the material to stretch uncontrollably, complicating the processing.

Preferably, the elastic film is formed from two non-elastic film layers coextruded with the elastic core layer and sandwiching it. The invention is not limited to any particular design of the elastic film.

Tables 1 and 2 show particularly preferred configurations of the elastic film 4 that have been used for the elastic laminate 1 in the examples.

**Table 1**

| **Layer / Type A** | **Thickness [µm]** | **Composition [wt%]** |
|---|---|---|
| **First non-elastic outer layer** | 3,5 | 57 % PE + 45 % PP + 8 % Talc |
| **Elastic film layer** | 28 | 80 % SBS + 12 % PS + 8 % Mineral Oil |
| **Second non-elastic outer layer** | 3,5 | 57 % PE + 45 % PP + 8 % Talc |

**Table 2**

| **Layer / Type B** | **Thickness [µm]** | **Composition [wt%]** |
|---|---|---|
| **First non-elastic outer layer** | 3 | 57 % PE + 45 % PP + 8 % Talc |
| **Elastic film layer** | 24 | 60 % POE + 40 % SEEPS |
| **Second non-elastic outer layer** | 3 | 57 % PE + 45 % PP + 8 % Talc |

The tables show that the non-elastic layers are significantly thinner than the elastic core layer, with an elastic film 4 of type A having an elastic core layer of 28 µm and non-elastic outer layers of 3.5 µm. In contrast, the elastic film 4 of type B is generally thinner, with the non-elastic outer layers being 3 µm thick and the elastic core layer 24 µm thick.

The non-elastic outer layers consist mainly of a polyolefin, with a polyolefin blend of 57% polyethylene and 35% polypropylene being used in both examples. In addition, 8% talc is present in the non-elastic outer layers.

The elastic core layers differ between type A and type B in that a styrene-butadiene-styrene copolymer (SBS) and 12% polystyrene were used for type A. In addition, 8% mineral oil is found in the elastic core layer, which mainly serves as a processing agent.

According to Type B, the elastic core layer is formed from 60% of a polyolefin blend and 40% of a styrene-ethylene-propylene-styrene block copolymer (SEEPS). Processing aids are not required in this context due to the good miscibility between SEEPS and a polyolefin.

### Manufacturing process

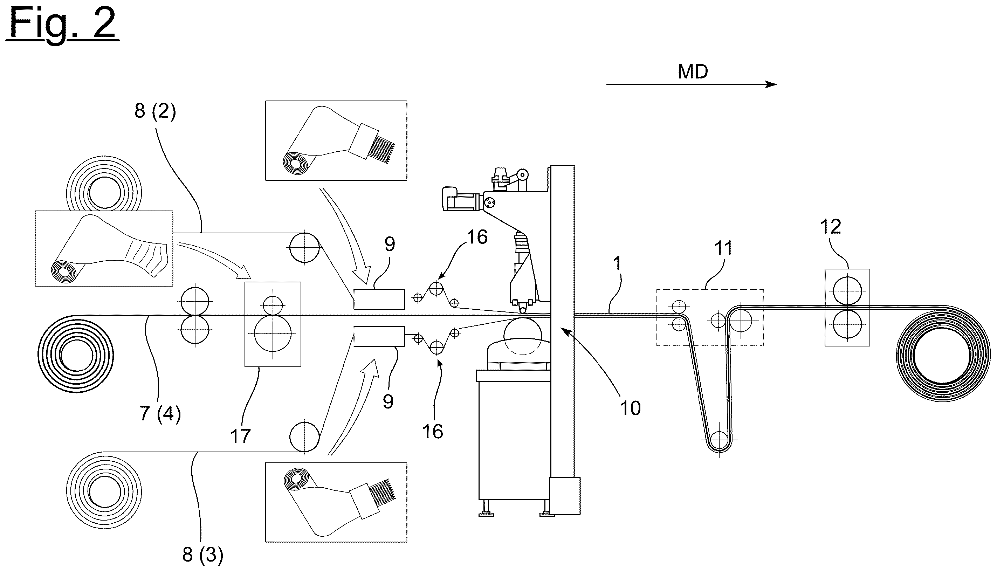

Fig. 2 shows a schematic representation of the manufacturing process for forming an elastic laminate 1 according to Fig. 1.

In a first step, the elastic film4 is supplied in the form of an elastic film web 7. The top layers 2, 3 are also each supplied as a nonwoven web 8 and moved together with the elastic film web 7 in the machine direction MD.

The nonwoven webs 8 are then each fed to a folding device 9, which pre-folds the nonwoven webs 8 in the cross-direction CD. This pre-folding process is indicated in the detail boxes, whereby in the course of the pre-folding process the width of the nonwoven web 8 is converted from a width in the original state A to a width in the folded state A'. The ratio between the width in the folded state A' and the width in the unfolded state A is preferably between 0.4 and 0.9.

The nonwoven webs 8 that have already been pre-folded are then stretched in in a first stretching-device 16 in the machine direction MD. This is essentially achieved by different roll speeds. Due to these different speeds, the nonwoven webs 8 that have already been pre-folded are stretched by at least 10 %. This ensures that the initially very roughly formed pre-folds are converted into micro-folds 5b which are shown in Fig. 5A.. At the same time, the web tension that causes the stretching ensures that the nonwoven webs 8 that have been folded also remain in the folded state and do not fall apart.

The elastic film web 7 is fed to a second stretching device 17, in which the elastic film web 7 is stretched in machine direction MD.

Then the nonwoven webs 8 folded into folds 5 are joined to the elastic film web 7 stretched in the machine direction MD in the joining device 10. This is advantageously done by means of so-called ultrasonic welding. In this case, a pattern of bonding points 6 is generated by means of a so-called anvil roller, via which the nonwoven webs 8 and elastic film web 7 are bonded to one another. In the present case, the elastic film web 8 is preferably melted so that the two nonwoven webs 8 are then welded directly to one another. This also causes openings to form at the bonding points 6, which results in a particularly high level of air permeability.

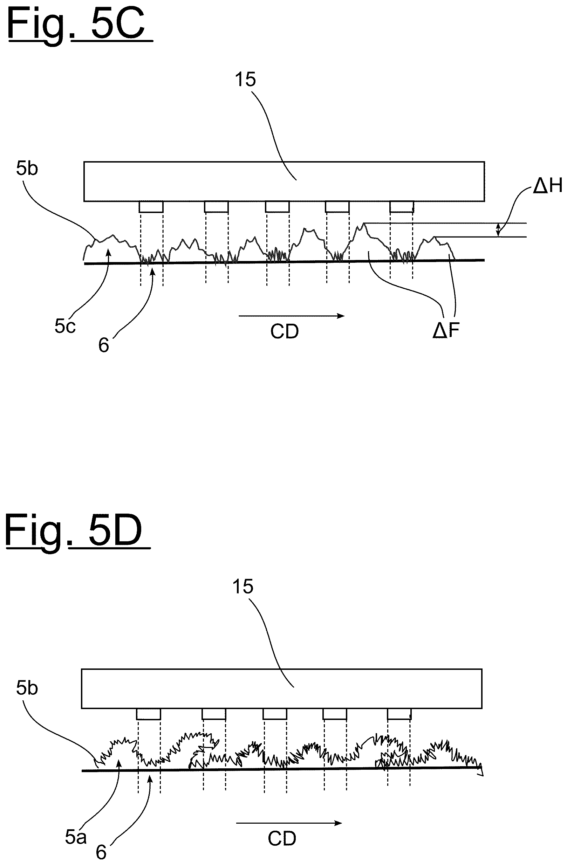

In this context, it is important to note that the position of the structure of the folds 5a after the folding device 9 is not specifically assigned to the position of the bonding points 6. Therefore, according to Fig. 5D, it is possible that a plurality of mico-folds 5b form a macro-structure 5a, which can also be overfolded. The bonding points 6 can then be introduced in areas where overfolding has taken place.

The overfolding has the consequence that in the finished laminate 1 individual macro-folds 5c between the bonding points 6 differ in their cross-sectional area other macro-folds 5c. These differences being noticeable in a difference in the area F or in the height H of the macro-folds 5c outside of the bonding points 6 according to Fig. 5C.

According to Fig. 5B the micro-folds 5b outside the bonding points 6 together form a macro-fold 5c. Accordingly, the folds 5 which run in machine direction MD and which are trapped in a bonding point 6 are designed as micro-folds 5b, wherein the folds 5 that are not trapped in a bonding point 6 are designed as macro-folds 5c, due to an unfolding of the micro-folds 5b.

A tension control unit 11 can be used to adjust the web tension in a controlled manner.

At the same time, an activation device 12 can be used to activate the elastic laminate 1 in the cross direction CD while being still stretched in MD-direction. This serves in particular to expose the holes at the bonding points 6. At the same time, it can also be used to cause individual micro-folds 5b to be pulled out of the bonding points6. Activating the laminate 1 (Fig. 5C and 7B) in the cross-direction pulls individual micro-folds 5b out of the bonding points 6. Alternatively, it is also conceivable that the micro-folds 5b tear or expand in the area of the bonding points 6. These micro-folds results in the folds 5c outside of the bonding areas 6 having an irregular cross-sectional shape (Fig. 5C), which has already been explained. At the same time, the width Wᵤ' of the area between the bonding points 6 is extended compared to the non-activated laminate 1. The combination of the folding device 9, the bonding device 10 and the activation device 12 allows the elasticity of the elastic laminate 1 to be adjusted in a targeted manner.

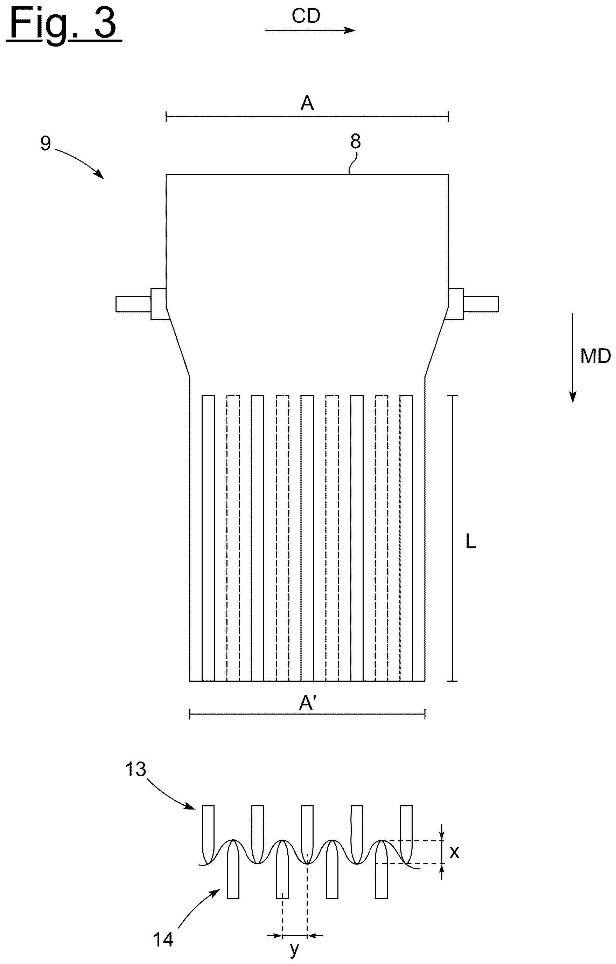

Of particular importance in this context is the folding device 9, which is explained in more detail in Fig. 3 in a schematic representation. In contrast to the solutions known from the prior art, the nonwoven webs 8 are pre-folded, whereby the pre-folds are subsequently converted into folds 5 in the form of micro-folds 5b. The pre-folding results in a particularly even distribution of the folds 5.

For this purpose, the nonwoven web 8 is supplied to the folding device 9 and the nonwoven web 8 is guided between an upper and a lower row of fins 13, 14, the fins 13, 14 guiding the nonwoven web 8 along a longitudinal section L in the machine direction MD in a supported manner. The length of the longitudinal section L is preferably 800 mm. The length is crucial for ensuring that a corresponding folding can take place between the upper and lower rows 13, 14 of fins. The upper row 13 and the lower row 14 of fins are preferably arranged in relation to one another in such a way that they interlock, with the dimension of the interlocking x preferably being between 5 and 50 mm. The distance between two spaced fins y is preferably between 5 and 40 mm.

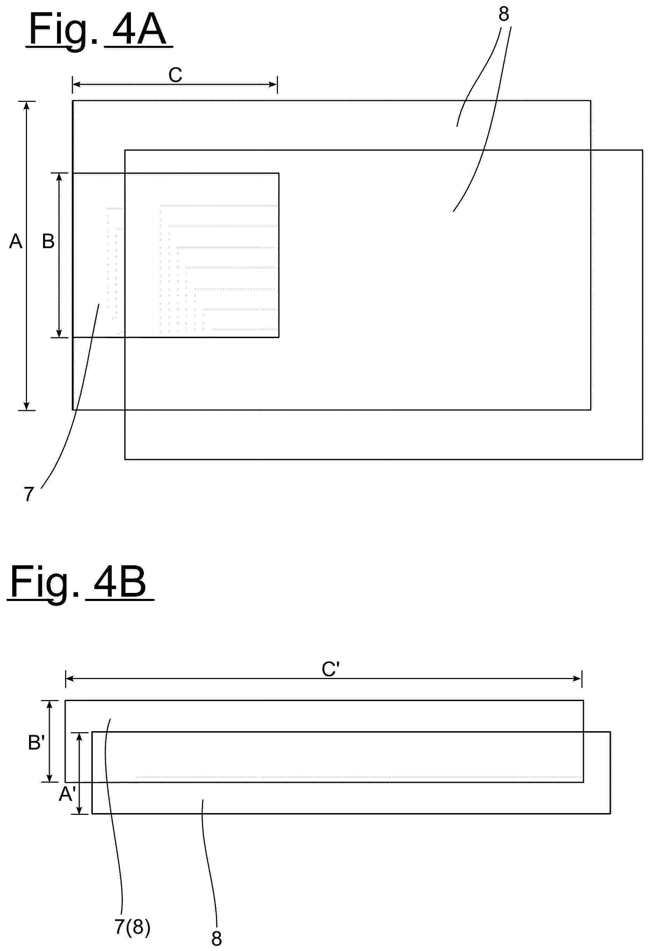

Figures 4A, 4B show the nonwoven web 8 and the elastic film web 7 in an unprocessed and in a processed state.

According to Figure 4A, the nonwoven webs 8 and the elastic film web 7 are supplied, the nonwoven webs 8 having a width A in an unprocessed state and the elastic film web having a width B. The ratio between the width A of the nonwoven web 8 and the width B of the elastic film is preferably between 0.8 and 4.0.

The nonwoven webs 8 are then folded, whereby the width is reduced to width A'. The ratio between the folded width A' of the nonwoven webs 8 and the width B of the as yet unprocessed film web 7 is between 0.4 and 0.9.

On the basis of Figure 4B, it is also clearthatthe elastic film web 7 in the stretched state also has a width B', which corresponds to the width A' of the nonwoven web 8 that has been laid in folds. This reduction in width B is achieved exclusively by stretching the elastic film web 7 in the machine direction MD from a length C to a length C' in the stretched state. The stretch corresponds to a value between 150% and 500%.

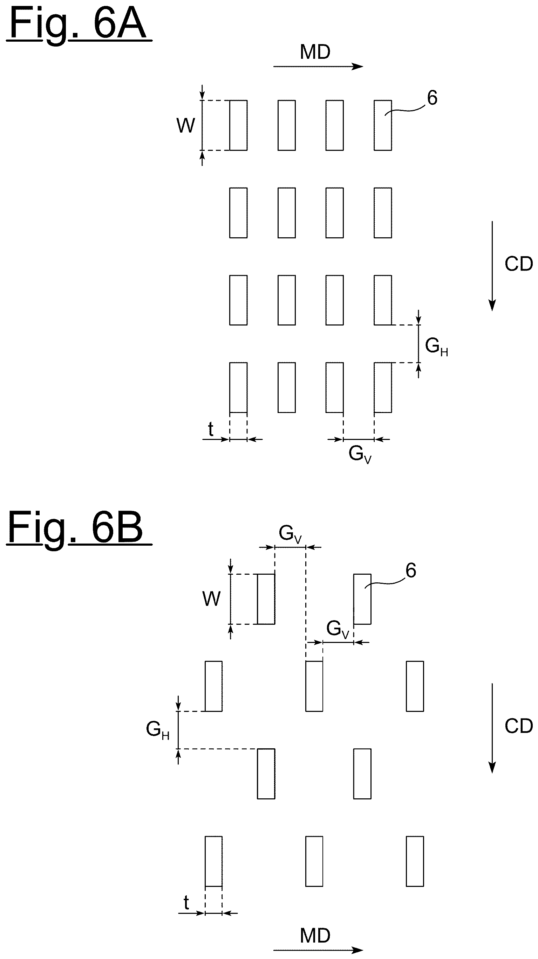

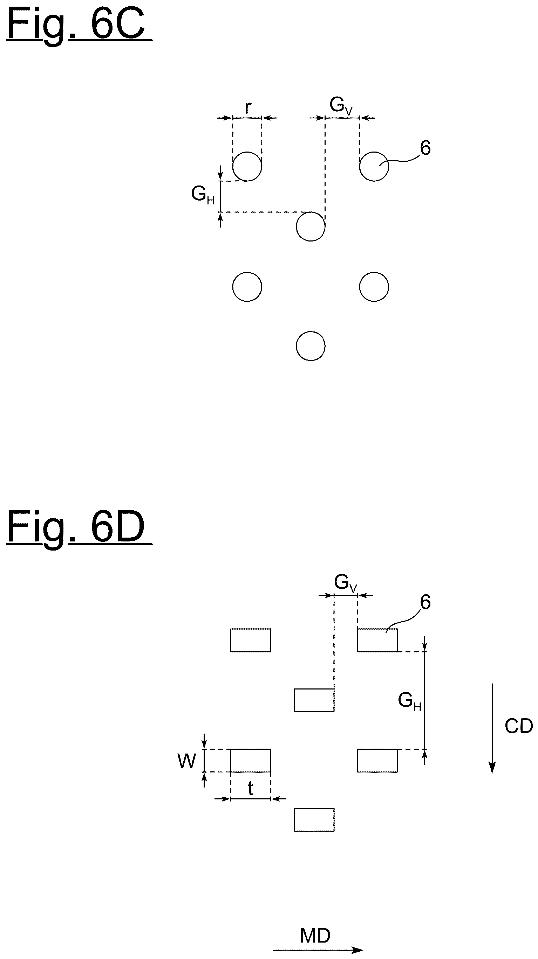

Figs. 6A, 6B, 6C, 6D show various patterns of bonding points 6 that can be used within the bonding device 10 to secure the nonwoven web 8 and the elastic film web 7.

Figure 6A shows rectangular bonding points 6, which are arranged regularly in both the machine direction MD and the cross-direction CD. Accordingly, bonding points6 are provided at all positionsin the machine direction MD and in the cross-direction CD. These bonding points 6 have a width W in the cross-direction CD and a depth T in the machine direction MD. Such a design has the advantage that it allows particularly uniform stretching in the cross-direction CD.

According to Fig. 6B, the bonding points 6 are arranged offset to one another in relation to the design in Fig. 6A, with only every second position occupied by a bonding point 6 in both the machine direction MD and the cross-direction CD.

Figure 6D is based on Figure 6B, with the bonding points 6 now having a greater depth than width W.

According to Figure 6C, the bonding points are circular.

### Examples

Tables 3 and 4 show various elastic laminates that differ from one another in terms of the materials used for the top layers 2, 3 and the elastic film4, the folding of the top layers 2, 3, the stretching of the elastic film 4 and any activation in the cross-direction.

Table 5 shows that all laminates have a very high elasticity, which isatleast219% in the machine direction (MD) and at least 131% in the cross-direction (CD). In addition, the elasticity in the cross-direction (CD) can be significantly increased by cross-activation of the laminate (Ex. 9 and 11), as a comparison with Ex. 8 and 10 shows.

The laminate is also characterized by a high tensile strength, which, based on the examples, is at least more than 36 in the machine direction (MD) and at least more than 27 N in the cross-direction (CD). The permanent set is also comparatively low, at a maximum of 23% in the machine direction (MD) and a maximum of 23% in the cross-direction (CD).

**Tabelle 3**

| | **Ex. 1** | **Ex. 2** | **Ex. 3** | **Ex. 4** | **Ex. 5** | **Ex. 6** |
|---|---|---|---|---|---|---|
| **Outer Layer 2** | Spunbond 10 gsm | Spunbond 10 gsm | Spunbond 10 gsm | Spunbond 10 gsm | Spunbond 8 gsm | Spunbond BiCo 13 gsm |
| **Elastic Layer 4** | Type A | Type A | Type B | Type A | Type A | Type A |
| **Outer Layer 3** | Spunbond 10 gsm | Spunbond 10 gsm | Spunbond 10 gsm | Spunbond 10 gsm | Spunbond 8 gsm | Spunbond BiCo 13 gsm |

| **Bonding Pattern** | | | | | | |
|---|---|---|---|---|---|---|
| **Type** | Fig. 5A | Fig. 5A | Fig. 5A | Fig. 5A | Fig. 5A | Fig. 5A |
| **W** | 1,4 mm | 1,4 mm | 1,4 mm | 1,4 mm | 1,4 mm | 1,4 mm |
| **t** | 0,3 mm | 0,3 mm | 0,3 mm | 0,3 mm | 0,3 mm | 0,3 mm |
| **Gv** | 1,75 mm | 1,75 mm | 1,75 mm | 1,75 mm | 1,75 mm | 1,75 mm |
| **G_{H}** | 1,0 mm | 1,0 mm | 1,0 mm | 1,0 mm | 1,0 mm | 1,0 mm |

| **Nonwoven Corrugation Process [mm]** | | | | | | |
|---|---|---|---|---|---|---|
| **A'/A** | 0,45 - 0,78 | 0,45 - 0,78 | 0,45 - 0,78 | 0,39 - 0,67 | 0,45 - 0,78 | 0,45 - 0,78 |
| **B'/B** | 0,52 - 0,92 | 0,52 - 0,92 | 0,52 - 0,92 | 0,46 - 0,79 | 0,52 - 0,92 | 0,52 - 0,92 |
| **C'/C** | 2,5 - 3,5 | 2,8 - 3,8 | 3 - 4,2 | 3 - 4,2 | 3 - 4,2 | 3 - 4,2 |

| **CD - Activation of the Laminate** | | | | | | |
|---|---|---|---|---|---|---|
| **x** | - | - | - | - | - | - |

**Tabelle 4**

| | **Ex. 7** | **Ex. 8** | **Ex. 9** | **Ex. 10** | **Ex. 11** |
|---|---|---|---|---|---|
| **Outer Layer 2** | Spunbond BiCo 13 gsm | Spunbond 10 gsm | Spunbond 10 gsm | Spunbond 10 gsm | Spunbond 10 gsm |
| **Elastic Layer 4** | Type A | Type A | Type B | Type A | Type A |
| **Outer Layer 3** | Spunbond BiCo 13 gsm | Spunbond 10 gsm | Spunbond 10 gsm | Spunbond 10 gsm | Spunbond 10 gsm |

| **Bonding Pattern** | | | | | |
|---|---|---|---|---|---|
| **Type** | Fig. 5A | Fig. 5A | Fig. 5A | Fig. 5A | Fig. 5A |
| **W** | 1,4 mm | 1,4 mm | 1,4 mm | 1,4 mm | 1,4 mm |
| **t** | 0,3 mm | 0,3 mm | 0,3 mm | 0,3 mm | 0,3 mm |
| **G_{V}** | 1,75 mm | 1,75 mm | 1,75 mm | 1,75 mm | 1,75 mm |
| **G_{H}** | 1,0 mm | 1,0 mm | 1,0 mm | 1,0 mm | 1,0 mm |

| **Nonwoven Corrugation Process [mm]** | | | | | |
|---|---|---|---|---|---|
| **A'/A** | 0,45 - 0,78 | 0,45 - 0,78 | 0,45 - 0,78 | 0,39 - 0,67 | 0,39 - 0,67 |
| **B'/B** | 0,52 - 0,92 | 0,52 - 0,92 | 0,52 - 0,92 | 0,46 - 0,79 | 0,46 - 0,79 |
| **C'/C** | 3 - 4,2 | 3 - 4,2 | 3 - 4,2 | 3 -4,2 | 3 - 4,2 |

| **CD -Activation of the Laminate** | | | | | |
|---|---|---|---|---|---|
| **x** | - | - | 4, 5 mm | - | 4, 5 mm |

**Tabelle 5**

| | **Ex. 1** | **Ex. 2** | **Ex. 3** | **Ex. 4** | **Ex. 5** | **Ex. 6** | **Ex. 7** | **Ex. 8** | **Ex. 9** | **Ex. 10** | **Ex. 11** |
|---|---|---|---|---|---|---|---|---|---|---|---|
| **Extensibility [%] (MD)** | 219 | 263 | 311 | 278 | 236 | 260 | 259 | 263 | 262 | 320 | 317 |
| **Extensibility [%] (CD)** | 150 | 153 | 150 | 152 | 156 | 144 | 146 | 144 | 215 | 131 | 152 |
| **Air Permeability (rel.) [m³/(m²*min]** | 11 | 15 | 20 | 18 | 20 | 21 | 11 | 14 | 28 | 20 | 27 |
| **Air Permeability (str.¹) [m³/(m²*min]** | 41 | 46 | 50 | 41 | 47 | 48 | 31 | 48 | 59 | 56 | 66 |
| **Basic Weight [gsm]** | 79 | 86,7 | 94,9 | 84 | 79,2 | 116 | 102,9 | 87,9 | 81 | 91,5 | 81,6 |
| **Tensile Strength (MD) [N]** | 50 | 49 | 49 | 53 | 72 | 91 | 73 | 51 | 40 | 45 | 36 |
| **Tensile Strength (CD) [N]** | 27 | 27 | 31 | 28 | 27 | 47 | 37 | 31 | 29 | 35 | 34 |
| **Permanent Set (MD)² [%]** | 23 | 15 | 10 | 15 | 16 | 15 | 16 | 17 | 15 | 12 | 11 |
| **Permanent Set (CD)³ [%]** | 18 | 18 | 20 | 22 | 16 | 23 | 18 | 18 | 5 | 21 | 13 |

| | | | | | | | | | | | |
|---|---|---|---|---|---|---|---|---|---|---|---|
| ¹ Stretch: 100 % ² Stretch: 150 % ³ Stretch: 70 % | | | | | | | | | | | |

## Claims

1. Elastic laminate with at least one nonwoven top layer and an elastic film bonded to it, **characterized in that** the elongation at a load of 700 g according to DIN ISO 527 is at least 150% in the machine direction and at least 100% in the cross-direction.

2. Elastic laminate according to claim 1, **characterized in that** the elongation ata load of 700 g according to DIN ISO 527 is at least200% in the machine direction and/or at least 140% in the cross-direction.

3. Elastic laminate according to claim 1 or 2, **characterized in that** the elongation at a load of 700 g according to DIN ISO 527 in a diagonal direction arranged at 45° to the machine direction is at least 100%.

4. Elastic laminate according to one of the preceding claims, **characterized in that** the elastic film and the at least one top layer are attached to one another at a multiplicity of spaced-apart bonding points.

5. Elastic laminate according to claim 4, **characterized in that** the at least one top layer has an undulating surface in an unstretched state, wherein the surface weight at the bonding points is greater than the surface weight between the bonding points.

6. Elastic laminate according to claim 5, **characterized in that** the surface weight in the stretched state at the bonding points is at least 10% greater than the surface weight between the bonding points.

7. Elastic laminate according to one of the preceding claims, **characterized in that** the tensile strength according to DIN ISO 527 in the machine direction is at least 35 N, preferably 40 N, and/or in the cross-direction is at least 25 N.

8. Elastic laminate according to one of the preceding claims, **characterized in that** the permanent set after an initial stretch of 150% in the machine direction is at most 30% and/or after an initial stretch of 70% in the cross-direction is at most 30%.

9. Elastic laminate according to one of the preceding claims, **characterized in that** the permanent set after an initial stretch of 150% in the machine direction is at most 20% and/or after an initial stretch of 70% in the cross-direction is at most 20%.

10. Elastic laminate according to one of the preceding claims, **characterized in that** the elastic film is arranged between two nonwoven top layers.

11. Elastic laminate according to claim 10, **characterized in that** the nonwoven top layers are at least partially bonded directly to one another at the bonding points.

12. Elastic laminate according to claim 10 or 11, **characterized in that** the bonding points are formed by ultrasonic welding.

13. Elastic laminate according to one of the preceding claims 13 to 15, **characterized in that** the bonding points are round, rectangular and/or oval.

14. Elastic laminate according to one of the preceding claims, **characterized in that** the elastic film has an elastic core layer and at least a first non-elastic outer layer coextruded with the elastic core layer.

15. Elastic laminate according to claim 14, **characterized in that** a second non-elastic outer layer is coextruded with the elastic core layer, the elastic core layer being arranged between the first and the second outer layer.

16. Elastic laminate according to one of the preceding claims, **characterized in that** the air permeability according to DIN EN ISO 9237 at a differential pressure of 100 Pa perpendicular to a surface of 20 cm² in the unstretched state is more than 10 m³/(m²*min) and/or in a state stretched by 100% in both the machine and cross-direction is more than 40 m³/(m²*min).

17. A method for producing an elastic laminate comprising at least the following steps:
a) supplying at least one nonwoven web and one elastic film web in a machine direction,
b) folding at least one nonwoven web in the cross-direction,
c) stretching the elastic film web in the machine direction, and
d) forming the elastic laminate by joining the stretched elastic film to the at least one nonwoven web that has been folded.

18. A method according to claim 17, wherein during step b) the pre-folding of the at least one web in cross-direction occurs while guiding the nonwoven web in a supported manner along a longitudinal section.

19. A method according to claim 17 or 18, wherein the at least one nonwoven web is stretched in the machine direction by at least 10% between steps b) and d).

20. A method according to any one of claims 17 to 19, wherein the nonwoven web during step b) is guided between an upper and a lower row of fins extending in the machine direction along the longitudinal section, the fins of the upper row engaging between fins of the lower row.

21. A method according to any one of claims 17 to 20, wherein the nonwoven web that has been folded has a cross-direction width that is between 40% and 90% of the unfolded nonwoven web.

22. A method according to any one of claims 17 to 21, wherein the elastic film is stretched between 150% and 500% in step d).

23. A method according to any one of claims 17 to 22, wherein the folds formed in step b) have a cross-direction width between 0.5 and 4 mm and/or a height between 0.3 and 2 mm.

24. A method according to any one of claims 17 to 23, wherein the elastic film and the at least one nonwoven web are bonded to one another at a multiplicity of spaced-apart bonding points in step d).

25. A method according to claim 24, wherein the bonding points are produced by ultrasonic welding.

26. A method according to claim 24 or 24, wherein the bonding points are arranged in longitudinal rows running in the machine direction and in transverse rows running in the cross-direction.

27. A method according to claim 26, wherein directly adjoining bonding points of a longitudinal row and/or a transverse row have different sizes.

28. A method according to claim 26 or 27, wherein the bonding points of two longitudinal rows adjoining one another in the cross-direction are arranged offset relative to one another in the machine direction.

29. A method according to any one of claims 24 to 28, wherein in step d) the bonding points each enclose a plurality of folds.

30. A method according to any one of claims 17 to 29, comprising a step e) following step d): activation of the elastic laminate in the cross-direction.

31. A method according to any one of claims 17 to 30, comprising a final step f): winding of the elastic laminate.

32. A method according to any one of claims 17 to 31, wherein at least two nonwoven webs are supplied and folded, the elastic film being arranged in the stretched state between the folded nonwoven webs and bonded thereto.

33. A method according to claim 32, wherein the nonwoven webs are bonded to one another through holes in the elastic film to form the bonding points.
